Europäisches Patentamt

European Patent Office

Office européen des brevets

(11) Publication number: **0 304 319 B1**

# EUROPEAN PATENT SPECIFICATION

(45) Date of publication of patent specification: **09.02.94**   (51) Int. Cl.⁵: **A61F  13/54**, A61F 13/46

(21) Application number: **88307707.5**

(22) Date of filing: **19.08.88**

(54) **Absorbent structures with gelling agent and absorbent articles containing such structures.**

(30) Priority: **19.08.87 US 87137**
**22.01.88 US 146904**

(43) Date of publication of application:
**22.02.89 Bulletin  89/08**

(45) Publication of the grant of the patent:
**09.02.94 Bulletin  94/06**

(84) Designated Contracting States:
**AT BE CH DE ES FR GB GR IT LI LU NL SE**

(56) References cited:
**EP-A- 0 156 649**
**EP-A- 0 202 126**
**US-A- 4 654 039**

(73) Proprietor: **THE PROCTER & GAMBLE COM-
PANY**
**One Procter & Gamble Plaza**
**Cincinnati Ohio 45202(US)**

(72) Inventor: **Goldman, Stephen A.**
**1260 Forest Court**
**Wyoming, OH 45215(US)**
Inventor: **Horney, James C.**
**3106 Lehman Road**
**Cincinnati, OH 45204(US)**
Inventor: **Retzsch, Herbert L.**
**7874 Sequia Court**
**Cincinnati, OH 45239(US)**

(74) Representative: **Bottema, Johan Jan et al**
**Procter & Gamble GmbH**
**Sulzbacher Strasse 40**
**D-65818 Schwalbach am Taunus (DE)**

**Description**

The present invention relates to fibrous web structures suitable for absorbing discharged body fluids, to an improvement in the common process for the manufacture of such structures, and to absorbent gelling agent compositions especially useful in these structures. Such structures can be incorporated into disposable absorbent articles such as sanitary napkins, infant diapers, adult incontinence pads and the like.

BACKGROUND OF THE INVENTION

The present invention relates to fibrous web structures suitable for absorbing discharged body fluids and to absorbent gelling agent compositions especially useful in these structures. Such structures can be incorporated into disposable absorbent articles such as sanitary napkins, infant diapers, adult incontinence pads and the like.

Absorbent structures which comprise entangled masses of fibers, i.e., fibrous webs, art well known in the art. Such structures can imbibe liquids, such as discharged body fluids, both by an absorption mechanism wherein fluid is taken up by the fiber material itself and by a wicking mechanism wherein fluid is acquired by, distributed through and stored in the capillary interstices between fibers. One means for improving the absorbency characteristics of such fibrous web structures is to incorporate therein so-called superabsorbent polymers which imbibe fluid to thereby form a swollen hydrogel material. The resulting hydrogel serves to retain fluid such as discharged body liquids within the structure. An absorbent structure of this type wherein hydrogel-forming materials in particulate (including fiber) form are incorporated into fibrous webs is disclosed in Weisman and Goldman; U.S. Patent 4,610,678; Issued September 9, 1986.

Prior to the date of the present invention, the size and configuration of particle-forming hydrogel-forming polymers (i.e. gelling agents) incorporated into absorbent structures have not been considered to be parameters which affect the absorption characteristics of the structures containing them, and wide ranges of particle sizes have been proposed. The above-cited U.S. Patent 4,610,678, for example, indicates that galling agent particles incorporated into such absorbent structures can range in size from 30 micrometers to 4 millimeters. In actual commercial practice, however, the particles of gelling agent employed in absorbent cores for disposable diapers are frequently irregular but not highly elongated in configuration, range in particle size from 45 to 850 micrometers and have a mass median particle size of from 200 to 370 micrometers. Particles of this shape and size are generally selected because of industrial hygiene and ease-of-processing considerations.

Some commercially available gelling agent materials are furthermore produced in the fort of particles which are agglomerates of smaller particles since the larger agglomerates are easier to handle in gelling agent synthesis and packaging operations. It is believed, however, that agglomerates of this type break down to some degree during their incorporation into disposable absorbent articles manufactured in commercial operations and during subsequent use of such articles. Products of this type such as diapers thus may actually contain gelling agent in the fort of particles that are somewhat smaller than the gelling agent agglomerates originally provided in raw material form for diaper-making operations.

Whatever the size and form of gelllng agent particles employed in known absorbent structures, the gelling agent is generally more expensive than the staple fiber component which forms the principal part of the structure. Accordingly, the materials, configurations and processing used in making such absorbent structures are often manipulated and adjusted in order to maximize the absorbent capacity of the absorbent structure and the effective absorbent capacity of the gelling agent material employed in the structure. In this manner, the minimum amount of gelling agent consistent with the realization of desired absorbent performance objectives can be used. This, in turn, tends to minimize the cost of producing the absorbent structure.

Given the fact that there is a continuing need to improve absorbent structure absorbency characteristics, it is an objective of the present invention to provide a type of absorbent structure configuration wherein structure absorbent capacity and effective capacity of the gelling agents therein can be improved.

It is a further object of this invention to provide such absorbent structures of improved absorbent capacity and gelling agent efficiency by the simple adjustment and control of particle size distribution of the gelling agent materials employed in the absorbent structure.

It is a further object of the present invention to provide gelling agent compositions which have controlled particle size characteristics and which are especially suitable for incorporation into such absorbent structures.

It is a further object of the present invention to provide disposable absorbent articles such as diapers, training pants, incontinence pads, sanitary napkins and the like, which utilize such absorbent structures of

2

improved absorbency characteristics to form their absorbent cores.

## SUMMARY OF THE INVENTION

According to one aspect of the present invention there is provided a liquid-absorbent structure comprising a web having a density of from 0.06 to 0.3 g/cm$^3$, said web comprising from 40% to 95% by weight of hydrophilic fibres and from 5% to 60% by weight of nonfibrous particles of hydrogel-forming gelling agent thoroughly dispersed among the hydrophilic fibres in the web, which polymeric gelling agent has an equilibrium gel volume of at least 20 grams of synthetic urine per gram of gelling agent, wherein when dispersed as aforesaid among the hydrophilic fibres in the web the said particles are discrete non-agglomerated particles or non fragile agglomerated particles, said particles having a mass median particle size in the range from 400 to 700 micrometers with no more than 16% by weight of these particles having a particle size less than 200 micrometers, and no more than 16% by weight of these particles having a particle size greater than 1200 micrometers, with the result that the effective equilibrium absorbent capacity of the said particles when in the said structure is greater than the corresponding effective equilibrium absorbent capacity of the particles in a comparative structure differing in composition ad construction from the first above-mentioned only in that the particles in the said comparative structure have a mass median particle size less than 400 micrometers.

According to another aspect of the present invention there is provided a composition especially suitable for use as an absorbent gelling agent in fibrous absorbent web structures, said composition comprising a plurality of nonfibrous, discrete non-agglomerated particles or non fragile agglomerated particles of a substantially water-insoluble, slightly crosslinked, partially neutralized, hydrogel-forming polymeric gelling agent material wherein:

(a) said polymeric gelling agent material comprises:

(i) from 50 mole percent to 99.999 mole percent of polymerized unsaturated, polymerizable, acid group containing monomers; and

(ii) from 0.001 mole percent to 5 mole percent of a crosslinking agent;

said polymeric gelling agent material having a degree of neutralization of at least 50% and a gel volume of at least 20 grams of synthetic urine per gram of gelling agent material; and

(b) said plurality of particles has a mass median particle size ranging from 400 to 700 micrometers, with no more than 16% by weight of said particles having a particle size less than 200 micrometers and no more than 16% by weight of said particles having a particle size greater than 1200 micrometers.

In a further aspect of the invention there are provided absorbent articles of improved absorbency characteristics. Such articles comprise a liquid impervious backing sheet, a liquid pervious topsheet and an absorbent core positioned between the backing sheet and the topsheet. The absorbent core comprises an absorbent web structure of the same type as hereinbefore described.

According to another aspect of the invention there is provided an improvement in the preferred known process for manufacturing absorbent structures wherein non-fibrous particles of hydogel-forming polymeric agent are fed from a source thereof which is periodically restocked, to be thoroughly dispersed among hydrophilic fibres, and a web is laid down from the said resultant thorough mixture of particles and fibres, the improvement comprising controlling the supply of particles at or from the said source thereof such that the particles are discrete non-agglomerated particles or nonfragile agglomerated particles and have a mass median particle size within certain defined limits.

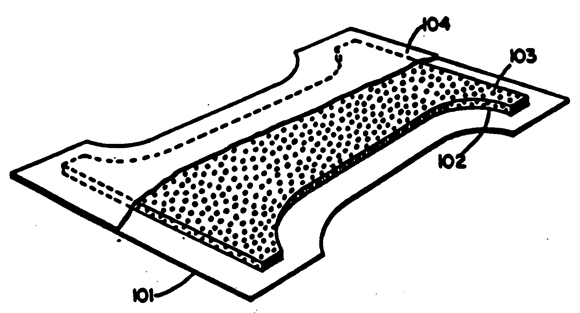

## BRIEF DESCRIPTION OF THE DRAWING

The drawing submitted herewith represents a cutaway view of a disposable diaper which is a preferred configuration for the absorbent articles herein.

## DETAILED DESCRIPTION OF THE INVENTION

The absorbent structures of the present invention are webs or batts which contain both fibrous and nonfibrous components, and the gelling agent compositions of the present invention contain only nonfibrous components. For purposes of this invention, the terms "fibers" and "fibrous" refer to a specific type of "particulate" material wherein the length to diameter ratio of the majority of such particulate material is greater than about 10. "Nonfibrous" particles, conversely, are those wherein the length to diameter ratio of the majority is about 10 or less.

In the first of its several embodiments, the present invention relates to absorbent structures which comprise fibrous webs containing nonfibrous particles of absorbent gelling agent material. The principal component of these absorbent web structures comprises hydrophilic fiber material. For purposes of the present invention, a fiber is "hydrophilic" if water or aqueous body fluid readily spreads on or over the surface of the fiber (without regard to whether or not the fiber actually imbibes fluid or forms a gel). The hydrophilic fibers which are employed will generally have an average diameter which ranges from 1 to 200 micrometers. More preferably, the average diameter of these hydrophilic fibers will range from 10 to 100 micrometers. Substantially all of the hydrophilic fibers incorporated into the structures herein preferably have a fiber length of at least 1 mm.

The type of hydrophilic fiber material is not critical for use in the structures of the present invention. Any type of hydrophilic fiber which is suitable for use in conventional absorbent products is also suitable for use in the absorbent structures herein. Examples of hydrophilic fiber material include cellulose, modified cellulose, rayon, polyesters such as polyethylene terephthalate (DACRON), hydrophilic nylon (HYDROFIL), and the like.

Suitable hydrophilic fibers also include hydrophobic fibers which have been hydrophilized with a hydrophilizing agent. Such fibers include surfactant-treated or silica-treated thermoplastic fibers derived, for example, from polyolefins such as polyethylene or polypropylene, polyacrylics, polyamides, polystyrenes, polyurethanes and the like. In fact, hydrophilized hydrophobic fibers which are in and of themselves not very absorbent and which, therefore, do not provide webs of sufficient absorbent capacity to be useful in conventional absorbent structures, are suitable for use in the structures of this invention by virtue of their good wicking properties. This is because, in the structures herein, wicking propensity of the fibers is far more important than the absorbent capacity of the fiber material itself.

For reasons of availability and cost, cellulosic fibers are generally preferred for use herein as the hydrophilic fiber component of the web structure. Most preferred are wood pulp fibers.

Other cellulosic fiber materials which may be especially useful in certain absorbent structures herein are the stiffened, twisted, curled, cellulosic fibers which can be produced by internally cross-linking cellulose fibers with a cross-linking agent. Fibers of this general type are disclosed, for example, in U.S. Patent Nos. 3224926, 3241553, 3440135, 3658613, 3932209 and 4035147.

One especially preferred type of stiffened, twisted, curled cellulose fibers useful as the hydrophilic fiber component of the absorbent web structures herein comprises cellulose fibers which have been internally crosslinked, for example with a $C_2$-$C_8$ dialdehyde, while such fibers are in a relatively dehydrated state. Such fibers can be defined in terms of their dry fiber and wet fiber twist counts (at least 4.5 twist nodes per millimeter dry and at least 0.5 twist nodes per millimeter less than that when wet and preferably also at least 3.0 twist nodes per millimeter wet) and by their fluid retention characteristics (average isopropyl alcohol retention value of less than 30%; average water retention value of from 28% to 50%). Stiffened, twisted, curled cellulosic fibers of this type art described in greater detail in European Patent Publication Nos. 251,676 and 252,650.

The hydrophilic fiber component of the structures herein will generally comprise from 40% to 95% by weight of the structure, more preferably from 60% to 95% by weight. Most preferably, the hydrophilic fiber component will comprise from 70% to 90% by weight of the structure. These concentrations of hydrophilic fiber material include the weight of hydrophilizing agent, if any, employed thereon.

The second essential component of the absorbent structures herein comprises discrete, nonfibrous, nonfragile particles of a particular type of polymeric gelling agent. These polymeric gelling agents are those materials which, upon contact with fluids (i.e., liquids) such as water or body fluids, imbibe such fluids and thereby form hydrogels. In this manner, fluid discharged into the absorbent structures herein can be acquired and held by the particles of the polymeric gelling agent, thereby providing the structures herein with enhanced absorbent capacity and/or improved fluid retention performance.

The polymeric gelling agent particles which are employed sill generally comprise a substantially water-insoluble, slightly cross-linked, partially neutralized, hydrogel-forming polymer material. Such polymer materials can be prepared from polymerizable, unsaturated, acid-containing monomers. Thus, such monomers include the olefinically unsaturated acids and anhydrides which contain at least one carbon to carbon olefinic double bond. More specifically, these monomers can be selected from olefinically unsaturated carboxylic acids and acid anhydrides, olefinically unsaturated sulfonic acids and mixtures thereof. Upon polymerization, monomeric units of this type will comprise from 50 mole percent to 99.999 mole percent, more preferably from 75 mole percent to 99.99 mole percent of the gelling agent material.

Suitable unsaturated acidic monomers for use in preparing the polymeric gelling agents used in this invention include those listed In Brandt/Goldman/Inglin; U.S. Patent 4,654,039, and in Reissue Patent No. 32649.

Preferred monomers include acrylic acid, methacrylic acid, and 2-acrylamido- 2-methyl propane sulfonic acid. Acrylic acid itself is especially preferred for preparation of the polymeric gelling agent material.

In the hydrogel-forming polymeric gelling agent the polymeric component formed from unsaturated, acid-containing monomers may be grafted on to other types of polymer moieties such as starch or cellulose. Acrylic acid grafted starch materials of this type are also especially preferred for use herein.

Preferred polymer gelling agents which can be prepared from conventional types of monomers include hydrolyzed acrylonitrile grafted starch, acrylic acid grafted starch, polyacrylates, maleic anhydride copolymers and combinations thereof. Especially preferred are the polyacrylates and acrylic acid grafted starch.

Whatever the nature of the basic polymer components of the hydrogel-forming polymeric gelling agents used in the absorbent structures herein, such materials will in general be slightly cross-linked. Cross-linking serves to render the hydrogel-forming polymer gelling agents used in this invention substantially water-insoluble, and cross-linking thus in part determines the gel volume and extractable polymer characteristics of the hydrogels formed from the polymeric gelling agents employed. Suitable cross-linking agents are well known in the art and include, for example, those described in greater detail in Masuda et al; U.S. Patent 4,076,663.

Preferred cross-linking agents are the di- or polyesters of unsaturated mono- or polycarboxylic acids with polyols, the bisacrylamides and the di- or triallyl amines. Especially preferred cross-linking agents are N,N'-methylenebisacrylamide, trimethylol propane triacrylate and triallyl amine. The cross-linking agent can generally comprise from about 0.001 mole percent to 5 mole percent of the resulting hydrogel-forming polymer material. More preferably, the cross-linking agent will comprise from 0.01 mole percent to 3 mole percent of the hydrogel-forming polymeric gelling agent used herein.

The slightly cross-linked, hydrogel-forming polymeric gelling agents which may be used in the structures of the present invention are generally employed in their partially neutralized form. For purposes of this invention, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to herein as the "degree of neutralization." Degree of neutralization will preferably not exceed 98%.

The polymeric gelling agent materials used in the absorbent structures herein must have a relatively high capacity for imbibing fluids encountered In absorbent structures. The absorbent capacity of such materials can be quantified by referencing the "gel volume" of the polymeric gelling agents which are to be selected for use in the present invention.

For purposes of this invention, gel volume can be defined in terms of the amount of synthetic urine absorbed by any given polymeric gelling agent and is specified as grams of synthetic urine per gram of polymeric gelling agent. Gel volume in synthetic urine can be determined by forming a suspension of 0.1-0.2 parts of dried polymeric gelling agent to be tested with 20 parts of synthetic urine. This suspension is maintained at ambient temperature under gentle stirring for a time sufficient, e.g., about 1 hour, for swelling equilibrium to be attained. Using a procedure described in greater detail hereinafter in the Test Methods section, the gel volume of the polymeric gelling agent in grams of synthetic urine per gram of polymeric gelling agent is then calculated from the weight fraction of the polymeric gelling agent in the suspension and the ratio of the liquid volume excluded from the formed hydrogel to the total volume of the suspension.

The structures of the present invention, and especially the structures which are to be used in diapers, adult incontinence products or training pants, will generally employ polymeric gelling agents having a gel volume of at least 20 grams of synthetic urine per gram of polymeric gelling agent. When the structures herein are constructed from cellulosic fibers such as wood pulp fibers, it may be desirable to utilize polymer gelling agent having a somewhat higher gel volume, i.e., a gel volume between 25 and 60 grams of synthetic urine per gram of gelling agent.

Structures constructed from certain types of cellulosic fiber material such as, for example, the stiffened, twisted, curled cellulosic fibers hereinbefore described may actually be more effective at absorbing fluid if gelling agents of somewhat lower gel volumes are employed. Gelling agent of generally lower gel volume tends to form hydrogels of generally higher gel strength (as quantified by hydrogel shear modulus in the manner described in the hereinbefore-referenced U.S. Patent 4.654.039 and U.S. Reissue Patent No. 32649. Thus, in structures wherein the hydrophilic fibers are stiffened, twisted, curled cellulose fibers, it may be preferable to employ polymeric gelling agent having a gel volume of from 20 to 35 grams of synthetic urine per gram of gelling agent.

Another feature of the polymeric gelling agents which are especially useful in the absorbent structures herein relates to the level of extractable polymer material present in such hydrogel-forming material. Extractable polymer levels can be determined by contacting a sample of hydrogel -forming polymeric gelling agent material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the polymeric gelling agents used herein is also set forth in the hereinbefore referenced U.S. Patent 4,654,039 and Reissue Patent No. 32649. Polymeric gelling agent materials especially useful in the structures herein are those which have an equilibrium extractables content in synthetic urine of no more than 17%, preferably no more than about 10% by weight of the polymeric gelling agent.

An essential feature of the present invention is the utilization of the above-described polymeric gelling agents in the structures herein in the form of nonfibrous, nonfragile particles having certain particle size characteristics. In particular, it has been discovered that an unexpected improvement in absorbent structure capacity and effective gelling agent capacity within an absorbent structure can be realized by incorporating polymeric gelling agent into such absorbent structures in the form of particles which are generally larger than those which have heretofore been conventionally employed. Generally, provision of gelling agent particles of this relatively larger size requires the use of one or more manufacturing or processing techniques which eliminate or reduce the amount of smaller, finer gelling agent particles which are introduced, along with hydrophilic fiber, into the absorbent structures herein. An upper limit on gelling agent particle size is also provided since gelling agent particles which are too large may be less desirable from a consumer aesthetics standpoint.

Specifically, the present invention requires the utilization in the absorbent structures herein of polymeric gelling agent particles of a selected mass median particle size and preferably a certain particle size deviation from the mass median particle size. For purposes of the present invention, particle size is defined as the dimension of a galling agent particle which is determined by sieve size analysis, Thus, for example, in principle, a particle that is retained on a sieve with 710 micrometer openings is considered to have a particle size' greater than 710 micrometers, a particle that passes through a sieve with 710 micrometers openings and is retained on a sieve with 500 micrometer openings is considered to have a particle size greater than 500 micrometers.

Further, for purposes of this invention, the mass median particle size of a given simple or plurality of galling agent particles is defined as the particle size which divides the sample or plurality in half on a mass basis, i.e., half of the sample or plurality by weight will have a particle size greater than the mass median particle size and half of the sample or plurality by weight will have a particle size less than the mass median particle size. Thus, for example, the mass median particle size of a sample of galling agent particles is 500 micrometers if one half of the sample by weight is retained on a sieve with openings of 500 micrometers. A standard particle-size plotting method (wherein cumulative weight percent of the particle sample retained on or passed through a given sieve size is plotted versus sieve-opening size on probability paper) is typically used to determine mass median particle size when the 50% mass value does not correspond to the size opening of a U.S.A. Standard Testing Sieve. A plot of this type is also typically used to determine the distribution of particle size about the mass median value.

The polymeric gelling agent particles employed in the absorbent structure of this invention must have a mass median particle size ranging from 400 to 700 micrometers with no more than 16% by weight of these gelling agent particles having a particle size less than 200 micrometers and no more than 16% by weight of these particles having a particle size greater than 1200 micrometers. More preferably, the mass median particle size of the gelling agent particles used in the structures herein will range from 410 to 650 micrometers with no more than 16% by weight of these particles having a particle size less than 210 micrometers and no more than 16% by weight of these particles having a particle size greater than 1000 micrometers. Most preferably, the mass median particle size of the gelling agent particles will range from about 420 to 600 micrometers with no more than 16% by weight of the particles having a particle size less than 220 micrometers and no more than 16% by weight of the particles having a particle size greater than 900 micrometers.

Within the foregoing mass median particle size and particle size distribution limitations, it is possible to even further identify preferred particle size characteristics for the gelling agent particles useful herein by means of standard sieve analyses. In a typical sieve analysis, a sample or plurality of gelling agent particles is sifted through a set number of screens of diminishing screen opening size and the weight percent of the sample retained on and/or passing through each screen is determined. Standard methods for making such sieve analyses have been established, for example, by the American Society for Testing Materials (ASTM).

One such method employs a Ro-Tap testing sieve shaker (manufactured by W. S. Tyler, Inc.) and a series of screens identified by either U.S. Sieve Series or Tyler Standard Sieve Series designations. Determination of particle size distribution using such a technique is described in greater detail in Perry's Chemical Engineers' Handbook, Sixth Edition, (McGraw-Hill Book Company, 1984) et pp. 21-13 to 21-19.

Particle size distribution characteristics as determined by a typical 5-screen ASTM standard analysis for preferred and more preferred gelling agent samples of the present invention are set forth as follows:

| Screen Opening Size (Micrometers) | U.S. Sieve Series Alternate Designation | Tyler Equivalent Designation | Preferred Distribution (Wt.%) | More Preferred Distribution (Wt.%) |
|---|---|---|---|---|
| 1410 | on No. 14 | on 12 mesh | < 0.1 | 0-0.05 |
| 1190 | on No. 16 | on 14 mesh | < 1.0 | 0-0.5 |
| 841 | on No. 20 | on 20 mesh | 10-35 | 15-25 |
| 297 | on No. 50 | on 48 mesh | > 50 | 60-70 |
| 149 | on No. 100 | on 100 mesh | < 15 | 10-14 |
| 149 | thru No. 100 | thru 100 mesh | ≦ 5 | 1-3 |

The polymeric gelling agent particles can be adjusted to, or close to, the requisite particle size distribution by controlling the processing techniques used to prepare the gelling agent. Frequently this will involve varying and monitoring the conditions under which the gelling agent is polymerized, dried, chopped, ground and/or agglomerated. Once gelling agent particles are formed by whatever process, further treatment such as screening may be required to remove particles which, if left in, would cause the gelling agent particle component to fall outside the scope of the hereinbefore-described particle size distribution requirements.

One preferred technique for preparing particles which are larger than those ordinarily prepared by gelling agent polymerization-drying-chopping techniques involves agglomeration of smaller particles to produce larger agglomerates. Agglomeration techniques can thus be used to raise the mass median particle size of gelling agent particles and to thereby provide particles in agglomerated form which are suitable for use in the structures herein. Agglomeration techniques are well known in the art and nay or may not involve the use of moisture addition to smaller gelling agent particles or the use of a binder or other type of agglomerating medium.

Gelling agent particles used in the process for the manufacture of the absorbent structures herein, whether or not in agglomerated form, should be non-fragile. For purposes of the present invention, such particles are nonfragile if they are stable enough to withstand the forces encountered in conventional absorbent structure manufacture and/or use without breaking apart and unduly separating into smaller component particles. This means that the gelling agent particles of the invention should be stable enough that they do not break apart into smaller particles to the extent that the resulting aggregation of particles (before swelling) falls outside the scope of the particle size limitations hereinbefore set forth. The nonfragility of a given batch of particles in any particular process for laying down a web of fibres comprising them, can be confirmed by, for example, sampling the resultant web in a manner statistically representative of the web structure as a whole, and carrying out a microscopic size analysis of the particles therein. To confirm that agglomerated particles are nonfragile at the point when contacted by body fluids, the behaviour of the agglomerates can be inspected under a microscope when contacted with representative volumes of synthetic urine. If the agglomerated particles break apart before being swollen with the synthetic urine, they will not be nonfragile for the purposes of this invention.

Polymeric gelling agent particles having the requisite particle size distribution are incorporated into the absorbent web structures of the present invention in a concentration of from 5% to 60% by weight of the structure. Use of gelling agent levels below 5% renders such structures less able to retain aqueous body fluids imbibed by the structure. At higher gelling agent concentrations, for example those above 60% by weight and even above 40% by weight, it is believed that the desirable effects of using generally larger gelling agent particles to improve absorbent capacity and effective gelling agent capacity wild diminish. Thus, preferably the gelling agent particles will comprise from 10% to 40% by weight of the absorbent structures herein.

The absorbent structures herein can contain a variety of optional materials in addition to the essential hydrophilic fiber and polymeric gelling agent components. Such optional materials can include, for example, fluid distribution aids, antimicrobials, pH control agents, perfumes, etc. If present, these optional components will generally comprise no more than 30% by weight of the structures herein.

The feature which distinguishes the process for manufacturing the absorbent structures of this invention from the preferred process of the prior art as represented by US-A-4610678 is the control of the supply of gelling agent particles to the mixing stage (wherein they are thoroughly dispersed with the hydrophilic fibres prior to laying down the web) such that the particles are discrete non-agglomerated particles or nonfragile agglomerated particles, and have the defined mass median particle size characteristics. Providing such control over the supply of gelling agent particle characteristics is maintained, the absorbent structures herein with their essential hydrophilic fiber and polymeric gelling agent particulate components can be prepared by any process or technique which provides a web comprising a combination of the fibers and the nonfibrous gelling agent particles. Within such a fiber/particle combination, the gelling agent particles should be thoroughly dispersed among the hydrophilic fibers forming the web. For purposes of this invention, gelling agent particles are "thoroughly dispersed" among the hydrophilic fibers if there are no significant instances of individual gelling agent particles being in contact only with each other and not with one or more hydrophilic fibers. Preferably, this will mean that there should be no zones or regions (e.g., zones or regions of 1 cubic centimeter or larger) within the absorbent structure wherein the concentration of gelling agent exceeds the 60% limit set forth hereinbefore.

Within the web structures herein, the particles of the polymeric gelling agent must be thoroughly dispersed but may or may not be uniformly distributed. In particular, there may be regions or zones of the web structures which have higher concentrations of gelling agent particles than do other regions or zones of the structure. In one embodiment of this type, there may be a concentration gradient of gelling agent particles along the thickness dimension of the structure with the lowest concentration of gelling agent being at or near the surface of the structure which receives fluid and the highest concentration of gelling agent being at or near the opposite end of the thickness dimension.

The density of the absorbent structures herein can be of some importance in determining the absorbent properties of the structures and of the absorbent articles in which such structures are employed. The density of the absorbent structures herein will generally be in the range of from 0.06 to 0.3 g/cm$^3$, and more preferably within the range of from 0.09 to 0.22 g/cm$^3$. Typically the basis weight of the absorbent structures herein can range from 0.02 to 0.12 g/cm$^2$.

Density values for these structures are calculated from basis weight and caliper. Caliper is measured under a confining pressure of 0.137 psi (0.94 kPa). Density and basis weight values include the weight of the hydrogel-forming particles. Density of the structures herein need not be uniform throughout the structures. Within the density ranges hereinbefore set forth, structures of this invention can contain regions or zones of relatively higher or relatively lower density.

Absorbent structures of this invention can be formed by air-laying a substantially dry mixture of hydrophilic fibers and polymeric gelling agent particles and, if desired or necessary, densifying the resulting web. Such a procedure is described more fully in Weisman and Goldman; U.S. Patent 4,610,678; Issued September 9, 1986,

As indicated in U.S. Patent 4,610,678, the air-laid webs formed by this procedure will preferably comprise substantially unbonded fibers and will preferably have a moisture content of 10% or less. In preparing webs of this invention by an air-laying process or by any other conventional procedure, care should be taken in handling and transporting the polymeric gelling agent particles so as to avoid breaking these particles down into smaller particles. This is especially true when the gelling agent particles are in an agglomerated form wherein the agglomerates may be more friable than nonagglomerated particles. If the gelling agent particles, e.g., agglomerates, are roughly treated during web structure preparation or thereafter, the particles actually incorporated into the structure (even though such particles are "nonfragile") may not have the requisite particle size distribution characteristics set forth hereinbefore.

In another of its embodiments, the present invention relates to gelling agent compositions per se which are especially suitable for incorporation as absorbents into fibrous absorbent web structures. Such compositions comprise a plurality of nonfibrous, nonfragile particles of polymeric gelling agent material. The gelling agent material itself is of the same type as hereinbefore described in detail. The plurality of gelling agent particles likewise has the same particle size characteristics as hereinbefore set forth for the gelling agent particles which are incorporated into the absorbent structure embodiments of this invention.

In yet another of its embodiments, the present invention relates to absorbent articles which utilize as an essential component an absorbent structure of the type hereinbefore described in detail. By "absorbent article" herein is meant a consumer product which is capable of absorbing significant quantities of water and other fluids (i.e., liquids), like body fluids. Examples of absorbent articles include disposable diapers, sanitary napkins, incontinence pads, paper towels, facial tissues, toilet pads and the like. The absorbent structures of this invention are particularly suitable for use in articles like diapers, incontinence products, and sanitary napkins.

Absorbent articles herein will in general comprise a liquid impervious backing sheet, a liquid pervious, relatively hydrophobic topsheet and an absorbent core comprising an absorbent structure of the present invention positioned between the backing sheet and the topsheet. Liquid impervious backing sheets can comprise any material, for example, polyethylene or polypropylene having a caliper of 0.037 mm (1.5 mils), which will help retain fluid within the absorbent article. Relatively hydrophobic, liquid pervious topsheets can comprise any material such as polyester, polyolefln, rayon and the like which is substantially porous and permits a fluid to readily pass therethrough into the underlying absorbent structure.

Particularly preferred absorbent articles herein are disposable diapers. Disposable diapers comprising the absorbent structures of the present invention may be made by using conventional diaper making techniques, but by replacing or supplementing the wood pulp fiber web ("airfelt") core which is typically used in conventional diapers with an absorbent structure of the present invention. Articles in the form of disposable diapers are fully described in U.S. Patent Nos. Re 26151, 3592194, 3489148 & 3860003.

A preferred disposable diaper for the purpose of this invention comprises an absorbent core containing an absorbent structure of this invention; a topsheet superposed or co-extensive with one face of the core, and a liquid impervious backsheet superposed or co-extensive with the face of the core opposite the face covered by the topsheet. The backsheet most preferably has a width greater than that of the core thereby providing side marginal portions of the backsheet which extend beyond the core. The diaper is preferably constructed in an hourglass configuration.

One embodiment of a disposable diaper article according to the present invention is shown in the drawing. The hourglass-shaped diaper structure of the drawing comprises a liquid impervious backing sheet 101. Positioned on the top of the backing sheet 101 is an hourglass-shaped absorbent core 102 comprising the absorbent structure of the present invention. This core contains hydrophillc fiber material such as wood pulp fiber. Also distributed throughout the absorbent core 102 are discrete particles 103 of polymeric gelling agent, which particles have the requisite size distribution hereinbefore described. Positioned on top of the hourglass-shaped absorbent core 102 is liquid pervious topsheet 104.

Because the absorbent structures of the present invention have a high absorbent capacity for menstrual fluid as well as for urine, such structures, even though defined in terms of capacity for synthetic urine, are also quite suitable for use in sanitary napkins. As is the case with disposable diapers, sanitary napkins utilizing the present absorbent structures may be derived from conventional sanitary napkins by simply replacing or supplementing the absorbent core thereof (typically a web of wood pulp fibers) with the gelling agent-containing absorbent structure of the present invention. Such replacement may be on a weight-by-weight basis, which results in a reduction in volume and a gain in capacity; or the replacement may be on a less than equal weight basis, thereby sacrificing part of the gain in absorbent capacity in favor of a reduction in bulk. The absorbent structures used in sanitary napkins preferably have a caliper of from 0.1 mm to 2 mm, more preferably from 0.3 mm to 1 mm.

An example of a sanitary napkin comprises a pad of the absorbent structure of the present invention; a hydrophobic topsheet; and a fluid impervious bottom sheet. The topsheet and the backsheet are placed at opposite sides of the absorbent structure. Optionally, the absorbent structure is wrapped in envelope tissue. Suitable materials for top sheets, bottom sheets and envelope tissue are well known in the art. A more detailed description of sanitary napkins and suitable materials for use therein is found in Duncan and Smith, U.S. Patent 3,871,378; Issued March 18, 1975.

TEST METHODS

In describing the present invention in the following examples, certain characteristics of the polymeric gelling agent particles and the absorbent structures containing them are set forth. Where reported, these characteristics can be determined using the following test methods:

Gel Volume of the Gelling Agent

Gel volume in terms of grams of synthetic urine absorbed per gram of polymeric gelling agent is determined by swelling the polymer samples in several aliquots of synthetic urine. The amount of such synthetic urine actually absorbed by the polymeric gelling agent is determined by a procedure which involves use of a synthetic urine solution containing Blue Dextran so that optical absorbance measurements can be used to calculate the amount of synthetic urine that is not taken up by the hydrogel which forms.

a) Blue Dextran Solution Preparation

A 0.03% Blue Dextran (BD) solution is prepared by dissolving 0.3 parts of Blue Dextran (Sigma D-5751) in 1000 parts of Synthetic Urine (SU) solution. Synthetic Urine is 15.0 parts of 1% TRITON X-100, 60.0 parts of NaCl, 1.8 parts of $CaCl_2 \cdot 2H_2O$, and 3.6 parts of $MgCl_2 \cdot 6H_2O$, diluted to 6000 parts with distilled $H_2O$. The resulting solution has an absorbonce of about 0.25 at its absorbance maximum of 617 nm.

b) Hydrogel Equilibration

Aliquots of the hydrogel-forming polymeric gelling agent to be tested are swelled in (i) 20 parts of Synthetic Urine (SU) solution and (ii) 20 parts of Blue Dextran (BD) solution. The suspension in the Blue Dextron (BD) solution is prepored in duplicate. For east hydrogels, 0.1 - 0.25 parts of hydrogel-forming dried powder is required to give a sufficiently high spectrophotometer reading relative to the Blue Dextran reference solution. One hour of equilibration at ambient temperature under gentle stir-bar stirring is usually sufficient for swelling equilibrium to be attained. After equilibration, a 3 ml aliquot of supernatant is separated from each gel suspension by decantation followed by centrifugation.

c) Gel Volume Determination

The optical absorbency (ABS) of each supernatant is determined spectrophotometrically with an accuracy of 0.001 absorbance units. The Synthetic Urine solution is used as an ABS = 0.0 reference. The absorbency of the supernatant from the synthetic urine suspension without Blue Dextran should not exceed 0.01 A; higher values indicate scattering from residual hydrogel gel particles or residual additives, and further centrifugation is necessary. The absorbency of the Blue Dextran supernatants should exceed the absorbency of the Blue Dextran reference solution by at least 0.1 absorbance units. Absorbency values below this range indicate the need to adjust the amount of polymeric gelling agent used to prepare the gel suspension.

d) Gel Volume Calculation

The Gel Volume in synthetic urine of the polymeric gelling agent in g/g is calculated from (i) the weight fraction of the polymeric gelling agent in the gel suspension and (ii) the ratio of the excluded volume to the total volume of the suspension. Since Blue Dextran is excluded from the hydrogel due to its high molecular weight, this ratio is related to the measured absorbencies. The following equation is used to calculate the gel volume:

$$\text{Gel Volume} = \left[ \frac{(g \quad \text{BD Solution})}{(g \quad \text{polymeric gelling agent*})} \right] \times \left[ 1 - \frac{(\text{ABS BD solution})}{(\text{ABS BD supernatant} - \text{ABS SU supernatant})} \right]$$

\* Corrected to a dry weight basis

Absorbent Structure Density

Reported density measurements are all made under a confining pressure of 9.6 $g/cm^2$ (0.94 kPa). Measurements are made using a Precision Scientific Penetrometer that is modified for caliper measurements.

Absorbent Capacity of Structure

The absorbent capacity of an absorbent structure can be determined by an equilibrium demand-absorbency test. In such a test, a 5.4 cm diameter sample of the absorbent structure is placed on a 7.5 cm diameter glass frit [Por E (ASTM 4-8 microns) from Ace Glass] that is maintained in fluid contact with a reservoir containing synthetic urine. In this setup, the reservoir is positioned on the pan of a Mettler AE160 Analytical Balance. This provides a continuous readout of the weight of synthetic urine in the reservoir and, by difference, the weight of synthetic urine taken up by the absorbent structure. The height of the frit and the height of the reservoir are adjusted to approximately the same level. A confining weight of 0.2 psi (1.38 kPa) is placed on top of the absorbent structure sample. Using this setup, the number of grams of synthetic urine taken up per gram of structure, once equilibrium is reached, can be determined.

EFFECTIVE EQUILIBRIUM ABSORBENT CAPACITY OF GELLING AGENT PARTICLES

This is the difference in grams between the absorbent capacity of a structure containing gelling agent particles, and that of a comparative structure differing in composition and construction from the first only in that the gelling agent particles are omitted, divided by the dry weight of the gelling agent particles.

The absorbent structures herein, as well as disposable absorbent articles containing them, are illustrated by the following examples.

EXAMPLE I

An absorbent structure with a basis weight of 0.072 g/cm$^2$ is prepared by airlaying a substantially uniform mixture of 80% by weight wood pulp fibers and 20% by weight of non-agglomerated particles of a cross-linked, partially neutralized, sodium polyacrylate gelling agent. The resulting fibrous web is then calendered to form a structure which has a density of 0.21 g/cm$^3$. The wood pulp fibers employed are southern soft wood slash pine fibers (Foley fluff). The gelling agent has an equilibrium gel volume of 24 grams of synthetic urine per gram of gelling agent and a degree of neutralization of 70%.

The particles of gelling agent are screened such that a No. 25/35 fraction is utilized in this absorbent structure. The 25/35 size fraction contains particles that pass through a U.S.A. Standard Testing Sieve with 710 micrometer openings (No. 25 U.S. Series Alternate Sieve Designation) and are retained on a U.S.A. Standard Testing Sieve with 500 micrometer openings (No. 35 U.S. Series Alternate Sieve Designation). This particle-size fraction is collected between U.S.A. Standard Testing Sieves (Fisher) after thirty minutes of sieving with a Ro-Tap Sieve Shaker (Tyler). The resultant particles have a mass median particle size between 500 and 710 micrometers.

The absorbent capacity of the structure containing gelling agent particles of this size fraction is determined using the procedure set forth hereinbefore in the Test Methods section and is found to be 11.4 grams of synthetic urine per gram of structure. Since it is known that the equilibrium capacity of a comparable 100% Foley fluff structure at 0.2 psi is about 6.0 g/g, it is possible to calculate an effective absorbent capacity of the gelling agent in the above structure. This is done by determining the capacity differential in grams between the gelling agent-containing structure and a comparable 100% Foley fluff structure and by dividing this difference by the dry weight in grams of the gelling agent. The effective absorbent capacity of the gelling agent determined in this manner thus represents the unit increase in equilibrium capacity of the absorbent structure per unit of added hydrogel-forming polymeric gelling agent. For purposes of this invention a comparable structure without the gelling agent is one which has the same basis weight and density as the Foley fluff component of the gelling agent-containing structure. Such a comparable structure for this Example I has a basis weight of 0.057 g/cm$^2$ and a density of 0.17 g/cm$^3$. Using this procedure for the Example I structure, the effective absorbent capacity of the gelling agent in the structure is calculated to be 33.0 grams of synthetic urine per gram of gelling agent.

EXAMPLE II

Absorbent structures similar to that of Example I with approximately the same concentration of gelling agent are prepared using different size fractions of the particles of the same type of gelling agent. These structures are also tested for their equilibrium absorbent capacities in the manner described in the Test Methods section. Furthermore, an effective absorbent capacity of the gelling agent in each structure is also calculated in a manner similar to that described in Example I.

A description of the products tested and absorbent capacity results are set forth in Table I.

Table I

| Effect of Polymeric Gelling Agent (PGA) Particle Size on Equilibrium Absorbent Capacities | | | |
|---|---|---|---|
| Structure No. | PGA Particle Size Range (micrometers) | Structure Absorbent Capacity (grams/gram) | Effective PGA Absorbent Capacity (grams/gram) |
| 1 | 500-710 | 11.5* | 33.3* |
| 2 | 355-500 | 11.2 | 31.3 |
| 3 | 180-250 | 10.6* | 28.5* |
| 4 | 125-180 | 9.9* | 25.0* |
| 5 | 90-125 | 9.7 | 23.8 |

*Average of two determinations

The Table I data indicate that Structures 1 and 2 (which contain particles having mass median particle sizes greater than 400 micrometers and less than 710 micrometers) of the present invention provide both a higher absorbent capacity for the structure and a higher effective absorbent capacity of the gelling agent than do similar structures (Structures 3-5) which utilize generally smaller gelling agent particles, having mass median particle sizes less than 400 micrometers.

EXAMPLE III

Absorbent structures similar to that of Examples I and II are prepared using the same components but with varying amounts and sizes of the gelling agent particles in the structures. Absorbent capacities of such structures and effective absorbent capacity of the gelling agent in each such structure are also determined. Such structures and absorbent capacities are described in Table II.

Table II

| Effect of Polymeric Gelling Agent (PGA) Concentration | | | | |
|---|---|---|---|---|
| Approximate PGA Concentration (Wt.%) | Structure Absorbent Capacity (grams/gram) | | Effective PGA Capacity (grams/gram) | |
| | Large[1] Particles | Small[2] Particles | Large[1] Particles | Small[2] Particles |
| 0 | 6.0* | 6.0* | -- | -- |
| 10 | 9.3* | 7.9* | 40* | 26* |
| 20 | 11.5* | 9.7 | 33* | 24 |
| 30 | 12.8* | 11.1* | 29* | 24* |

[1]500-710 micrometers
[2]90-125 micrometers
*Average of duplicate measurements

The Table II data indicate that the improvement in the effective absorbent capacity of the gelling agent, which improvement is brought about by using larger particle size gelling agent, diminishes as total gelling agent concentration in the structure increases.

EXAMPLE IV

An absorbent structure similar to that described in Example I is prepared using a cross-linked, sodium polyacrylate gelling agent having a gel volume of 49 grams of synthetic urine per gram and a degree of neutralization of 70%. The particle-size fraction of such a gelling agent is such that the gelling agent particles pass through a U.S.A. Standard Testing Sieve with 710 micrometer openings (No. 25 U.S. Series Alternate Sieve Designation) and are retained on a U.S.A. Standard Testing Sieve with 500 micrometer openings (No. 35 U.S. Series Alternate Sieve Designation). These gelling agent particles are incorporated into the Foley Fluff structure in a concentration of about 15%. The resulting fibrous web has a basis weight

12

of 0.05 g/cm$^2$. This web is then calendered to form a structure which has a density of 0.19 g/cm$^3$.

EXAMPLE V

The equilibrium absorbent capacity of, and the effective capacity of gelling agent in, the structure of Example IV are compared with those of similar structures wherein smaller particle size fractions of the gelling agent are employed. Results are shown in Table III.

Table III

| Effect of Polymeric Gelling Agent (PGA) Particle Size Using High Gel Volume PGAs | | | | |
|---|---|---|---|---|
| Structure No. | PGA Concentration (Wt%) | PGA Particle Size Range (micrometers) | Structure Absorbent Capacity (grams/gram) | Effective PGA Absorbent Capacity (grams/gram) |
| 1 | 15.2* | 500-710 | 12.2* | 46.6* |
| 2 | 14.5 | 355-500 | 12.1 | 48.0 |
| 3 | 14.6 | 180-250 | 11.1 | 41.3 |
| 4 | 13.0 | 125-180 | 10.3 | 39.4 |
| 5 | 14.5 | 90-125 | 10.0 | 33.5 |

*Average of two determinations

The Table III data indicate that, for this relatively high gel volume gelling agent, Structures 1 and 2 (which contain particles having mass median particle sizes greater than 400 micrometers and less than 710 micrometers) of the present invention provide both a higher absorbent capacity for the structure and a higher effective absorbent capacity of the the gelling agent than do similar structures (Structures 3-5) which utilize generally smaller gelling agent particles, having mass median particle sizes less than 400 micrometers.

EXAMPLE VI

A disposable diaper is prepared using an absorbent structure of the present invention as an absorbent core. The absorbent structure is an hourglass-shaped air-laid mixture comprising 26.6 grams of wood pulp fibers (Foley fluff) and 5 grams of particles of a cross-linked sodium polyacrylate polymeric gelling agent having a gel volume of 30 grams of synthetic urine per gram and a degree of neutralization of 70%. Substantially all of the particles of the polymeric gelling agent range in size from about 355-500 micrometers. Such particles have a mass median particle size of 430 micrometers.

The absorbent structure has nonuniform distribution of gelling agent therein such that the weight percent of gelling agent in the front section of the diaper is 20%, in the crotch section of the diaper is 13% and in the back section of the diaper is 12%. Such a structure is calendered to an average density of 0.15 g/cm$^3$ and has an average basis weight of 0.05 g/cm$^2$.

This hourglass-shaped absorbent structure is utilized as an absorbent core in a diaper prepared in general as described in Buell, U.S. Patent 3,860,003; Issued January 14, 1975. In such a diaper, the hourglass core is overwrapped with envelope tissue and inserted between a polypropylene topsheet and a polyethylene backing sheet. Such a diaper structure is especially effective at using the absorbent capacity of the gelling agent in comparison with similar diapers employing smaller particle size fractions of the gelling agent.

**Claims**

1. A liquid-absorbent structure comprising a web having a density of from 0.06 to 0.3 g/cm$^3$, said web comprising from 40% to 95% by weight of hydrophilic fibres and from 5% to 60% by weight of nonfibrous particles of hydrogel-forming gelling agent thoroughly dispersed among the hydrophilic fibres in the web, which polymeric gelling agent has an equilibrium gel volume of at least 20 grams of synthetic urine per gram of gelling agent,
   characterised in that when dispersed as aforesaid among the hydrophilic fibres in the web the said particles are discrete non-agglomerated particles or non fragile agglomerated particles, said particles

EP 0 304 319 B1

having a mass median particle size in the range from 400 to 700 micrometers with no more than 16% by weight of these particles having a particle size less than 200 micrometers, and no more than 16% by weight of these particles having a particle size greater than 1200 micrometers, with the result that the effective equilibrium absorbent capacity of the said particles when in the said structure is greater than the corresponding effective equilibrium absorbent capacity of the particles in a comparative structure differing in composition and construction from the first above-mentioned only in that the particles in the said comparative structure have a mass median particle size less than 400 micrometers.

2. A liquid-absorbent structure according to claim 1 characterised in that the said nonfibrous particles of gelling agent, when dispersed among the hydrophilic fibres in the web,
(a) have a mass median particle size in the range 410 to 650 micrometers with no more than 16% by weight of those particles having a particle size less than 210 micrometers and no more than 16% by weight having a particle size greater than 1000 micrometers; or
(b) have a mass median particle size in the range 420 to 600 micrometers with no more than 16% by weight of the particles having a particle size less than 220 micrometers and no more than 16% by weight having a particle size greater than 900 micrometers.

3. A liquid-absorbent structure according to either one of Claims 1 & 2 characterised in that the said web comprises from 70% to 90% by weight of the said hydrophilic fibres and from 10% to 30% by weight of the said nonfibrous particles of gelling agent.

4. A liquid-absorbent structure according to any of Claims 1 to 3 characterised in that the hydrophilic fibres are cellulosic fibres.

5. A liquid-absorbent structure according to any of Claims 1 to 4 characterised in that the hydrophilic fibres are wood pulp fibres and the hydrogel-forming polymeric gelling agent has a gel volume of 25 to 60 grams of synthetic urine per gram of gelling agent.

6. A liquid-absorbent structure according to any of Claims 1 to 4 characterised in that the hydrophilic fibres are stiffened, twisted, curled cellulose fibres and in that the hydrogel-forming polymeric gelling agent has a gel volume of from 20 to 35 grams of synthetic urine per gram of gelling agent.

7. A liquid-absorbent structure according to any of Claims 1 to 6 characterised in that the hydrogel-forming polymeric gelling agent is a slightly cross-linked, partially neutralised polymer selected from polyacrylates and acrylic acid grafted starch, said gelling agent having an equilibrium extractable polymer content in synthetic urine of no more than 17% by weight.

8. A liquid-absorbent article comprising:
(a) a liquid impervious backing sheet;
(b) a liquid pervious top sheet; and
(c) a liquid-absorbent core positioned between said backing sheet and said top sheet,
characterised in that said absorbent core comprises a liquid-absorbent structure according to any of Claims 1 to 7.

9. A process for the manufacture of a liquid-absorbent structure comprising a web having a density of from 0.06 to 0.3g/cm$^3$, said web comprising from 40% to 95% by weight of hydrophilic fibres and from 5% to 60% by weight of nonfibrous particles of hydrogel-forming polymeric gelling agent having an equilibrium gel volume of at least 20 grams of synthetic urine per gram of gelling agent, which process includes a step wherein particles of the said gelling agent are fed, from a source thereof which is periodically restocked, to be thoroughly dispersed among the said hydrophilic fibres and the said web is laid down from the resultant thorough mixture of fibres and particles, characterised in that the process comprises controlling the supply of the said particles at or from the said source thereof, such that the particles are discrete non-agglomerated particles or non fragile agglomerate particles having a median particle size in the range of 400 to 700 micrometers, with no more than 16% by weight of these particles having a particle size less than 200 micrometers and no more than 16% by weight of these particles having a particle size greater than 1200 micrometers, with the result that the effective equilibrium absorbent capacity of the said particles when in the said structure is greater than the corresponding equilibrium absorbent capacity of the particles in a comparative structure differing in

14

composition and construction from the first above-mentioned only in that the particles in the said comparative structure have a mass median particle size less than 400 micrometers.

10. A process according to Claim 9 <u>characterised in that</u> the supply of said nonfibrous particles of gelling agent from the said source is controlled such that the said particles:

(a) have a mass median particle size in the range 410 to 650 micrometers with no more than 16% by weight of those particles having a particle size less than 210 micrometers and no more than 16% by weight having a particle size greater than 1000 micrometers; or

(b) have a mass median particle size in the range 420 to 600 micrometers with no more than 16% by weight of the particles having a particle size less than 220 micrometers and no more than 16% by weight having a particle size greater than 900 micrometers.

11. A composition especially suitable for use as an absorbent gelling agent in fibrous absorbent web structures, said composition comprising a plurality of nonfibrous, discrete non-agglomerated particles or non fragile agglomerated particles of a substantially water-insoluble, slightly crosslinked, partially neutralized, hydrogel-forming polymeric gelling agent material wherein:

(a) said polymeric gelling agent material comprises:

(i) from 50 mole percent to 99.999 mole percent of polymerized unsaturated, polymerizable, acid group containing monomers; and

(ii) from 0.001 mole percent to 5 mole percent of a crosslinking agent;

said polymeric gelling agent material having a degree of neutralization of at least 50% and a gel volume of at least 20 grams of synthetic urine per gram of gelling agent material; and

(b) said plurality of particles has a mass median particle size ranging from 400 to 700 micrometers, with no more than 16% by weight of said particles having a particle size less than 200 micrometers and no more than 16% by weight of said particles having a particle size greater than 1200 micrometers.

12. A composition according to Claim 11 wherein the polymeric gelling agent material is selected form polyacrylates and acrylic acid grafted starch and has an equilibrium extractable polymer content in synthetic urine of no more than 17% by weight.

13. A composition according to Claim 12 wherein said polymer gelling agent material has a gel volume of from 25 to 60 grams of synthetic urine per gram of gelling agent.

14. A composition according to Claim 13 wherein said plurality of gelling agent particles has the following particle size distribution as determined by ASTM analysis on U.S. Sieve Series screens:

| U.S. Sieve Series Alternative Designation | Distribution (Wt%) |
|---|---|
| On No. 14 | Up to 0.1 |
| On No. 16 | Up to 1.0 |
| On No. 20 | 10-35 |
| On No. 50 | Less than 50 |
| On No. 100 | Up to 15 |
| Thru No. 100 | Less than or equal to 5 |

**Patentansprüche**

1. Eine flüssigkeitsabsorbierende Struktur, welche eine Bahn mit einer Dichte von 0.06 bis 0.3 g/cm$^3$ umfaßt, wobei die genannte Bahn 40 bis 95 Gew.-% hydrophile Fasern und 5 bis 60 Gew.-% unter den hydrophilen Fasern in der Bahn gründlich dispergierte nichtfaserige Partikel eines Hydrogel bildenden Geliermittels enthält, welches polymerische Geliermittel ein Gleichgewichtsgelvolumen von mindestens 20 g synthetischen Urin pro Gramm Geliermittel aufweist,

<u>dadurch gekennzeichnet</u>, daß die genannten Partikel, wenn sie, wie zuvor erwähnt, unter den hydrophilen Fasern in der Bahn dispergiert sind, einzelne nicht-agglomerierte Partikel oder nicht fragile agglomerierte Partikel sind, wobei die genannten Partikel eine Massemittelwert-Partikelgröße im Bereich von 400 bis 700 Mikrometer mit nicht mehr als 16 Gew.-% dieser Partikel mit einer Partikelgröße von weniger als 200 Mikrometer und nicht mehr als 16 Gew.-% dieser Partikel mit einer Partikelgröße von mehr als 1200 Mikrometer aufweisen, mit dem Ergebnis, daß die wirksame Gleichgewichtsabsorptionskapazität der genannten Partikel, wenn sie in der genannten Struktur sind, größer ist als die entsprechende wirksame Gleichgewichtsabsorptionskapazität der Partikel in einer Vergleichsstruktur, welche in Zusammensetzung und Konstruktion sich von der ersten zuvor erwähnten nur darin unterscheidet, daß die Partikel in der genannten Vergleichsstruktur eine Masse-Mittelwert-Partikelgröße von weniger als 400 Mikrometer aufweisen.

2. Eine flüssigkeitsabsorbierende Struktur nach Anspruch 1, dadurch gekennzeichnet, daß die genannten nichtfaserigen Partikel Geliermittels, wenn sie unter den hydrophilen Fasern in der Bahn dispergiert sind,

(a) eine Massemittelwert-Partikelgröße im Bereich von 410 bis 650 Mikrometer mit nicht mehr als 16 Gew.-% jener Partikel mit einer Partikelgröße von weniger als 210 Mikrometer und nicht mehr als 16 Gew.-% mit einer Partikelgröße von mehr als 1000 Mikrometer aufweisen; oder

(b) eine Massemittelwert-Partikelgröße im Bereich von 420 bis 600 Mikrometer mit nicht mehr als 16 Gew.-% der Partikel mit einer Partikelgröße von weniger als 220 Mikrometer und nicht mehr als 16 Gew.-% mit einer Partikelgröße von mehr als 900 Mikrometer aufweisen.

3. Eine flüssigkeitsabsorbierende Struktur nach einem der Ansprüche 1 und 2, dadurch gekennzeichnet, daß die genannte Bahn 70 bis 90 Gew.-% die genannten hydrophilen Fasern und 10 bis 30 Gew.-% die genannten nichtfaserigen Partikel Geliermittels umfaßt.

4. Eine flüssigkeitsabsorbierende Struktur nach einem der Ansprüche 1 bis 3, dadurch gekennzeichnet, daß die hydrophilen Fasern Zellulosefasern sind.

5. Eine flüssigkeitsabsorbierende Struktur nach einem der Ansprüche 1 bis 4, dadurch gekennzeichnet, daß die hydrophilen Fasern Zellstoff-Fasern sind und daß das Hydrogel bildende polymerische Geliermittel ein Gelvolumen von 25 bis 60 Gramm synthetischen Urins pro Gramm Geliermittel hat.

6. Eine flüssigkeitsabsorbierende Struktur nach einem der Ansprüche 1 bis 4, dadurch gekennzeichnet, daß die hydrophilen Fasern versteifte, gedrallte, gekräuselte Zellulosefasern sind und daß das Hydrogel bildende polymerische Geliermittel ein Gelvolumen von 20 bis 35 Gramm synthetischen Urins pro Gramm Geliermittel aufweist.

7. Eine flüssigkeitsabsorbierende Struktur nach einem der Ansprüche 1 bis 6, dadurch gekennzeichnet, daß das Hydrogel bildende polymerische Geliermittel ein schwach vernetztes, teilweise neutralisiertes Polymer, ausgewählt aus Polyacrylaten und Acrylsäure-gepfropfter Stärke, ist, wobei das genannte Geliermittel in synthetischem Urin einen Gleichgewichtsgehalt an extrahierbarem Polymer von nicht mehr als 17 Gew.-% aufweist.

8. Ein flüssigkeitsabsorbierender Artikel, welcher umfaßt:

(a) ein flüssigkeitsundurchlässiges Stützblatt;
(b) ein flüssigkeitsdurchlässiges Deckblatt; und
(c) einen flüssigkeitsabsorbierenden Kern, welcher zwischen dem genannten Stützblatt und dem genannten Deckblatt positioniert ist,

dadurch gekennzeichnet, daß der genannte absorbierende Kern eine flüssigkeitsabsorbierende Struktur nach einem der Ansprüche 1 bis 7 umfaßt.

9. Ein Verfahren zur Herstellung einer flüssigkeitsabsorbierenden Struktur, welche eine Bahn mit einer Dichte von 0.06 bis 0.3g/cm$^3$ umfaßt, wobei die genannte Bahn 40 bis 95 Gew.-% hydrophile Fasern und 5 bis 60 Gew.-% nichtfaserige Partikel Hydrogel bildenden polymerischen Geliermittels mit einem Gleichgewichtsgelvolumen von mindestens 20 Gramm synthetischen Urins pro Gramm Geliermittel umfaßt, welches Verfahren einen Schritt inkludiert, bei welchem Partikel des genannten Geliermittels von dessen Quelle, welche periodisch aufgefüllt wird, zugeführt werden, um unter den genannten hydrophilen Fasern vollständig dispergiert zu werden, und bei welchem die genannte Bahn von der resulierenden gründlichen Mischung aus Fasern und Partikeln abgelegt wird, dadurch gekennzeichnet, daß das Verfahren ein Kontrollieren des Zuführens der genannten Partikel an oder von deren genannter Quelle umfaßt, sodaß die Partikel einzelne nicht-agglomerierte Partikel oder nicht-fragile agglomerierte Partikel mit einer Mittelwert-Partikelgröße im Bereich von 400 bis 700 Mikrometer sind, mit nicht mehr als 16 Gew.-% dieser Partikel mit einer Partikelgröße von weniger als 200 Mikrometer und nicht mehr als 16 Gew.-% dieser Partikel mit einer Partikelgröße von mehr als 1200 Mikrometer, mit dem Ergebnis, daß die wirksame Gleichgewichtsabsorptionskapazität der genannten Partikel, wenn sie sich in der genannten Struktur befinden, größer ist als die korrespondierende Gleichgewichtsabsorptionskapazatät der Partikel in einer Vergleichsstruktur, welche sich in Zusammensetzung und Konstruktion von der ersten oben erwähnten nur dadurch unterscheidet, daß die Partikel in der genannten Vergleichsstruktur eine Massemittelwert-Partikelgröße von weniger als 400 Mikrometer aufweisen.

10. Ein Verfahren nach Anspruch 9, dadurch gekennzeichnet, daß das Zuführen der genannten nichtfaserigen Partikel Geliermittels von der genannten Quelle so kontrolliert wird, daß die genannten Partikel:
(a) eine Massemittelwert-Partikelgröße im Bereich von 410 bis 650 Mikrometer mit nicht mehr als 16 Gew.-% jener Partikel mit einer Partikelgröße von weniger als 210 Mikrometer und nicht mehr als 16 Gew.-% mit einer Partikelgröße von mehr als 1000 Mikrometer aufweisen; oder
(b) eine Massemittelwert-Partikelgröße im Bereich von 420 bis 600 Mikrometer mit nicht mehr als 16 Gew.-% der Partikel mit einer Partikelgröße von weniger als 220 Mikrometer und nicht mehr als 16 Gew.-% mit einer Partikelgröße von mehr als 900 Mikrometer aufweisen.

11. Eine Zusammensetzung, welche besonders geeignet zur Verwendung als ein absorbierendes Geliermittel in faserigen absorbierenden Bahnstrukturen ist, wobei die genannte Zusammensetzung eine Mehrzahl von nichtfaserigen, einzelnen nichtagglomerierten Partikel oder nicht-fragilen agglomerierten Partikeln eines im wesentlichen wasserunlöslichen, schwach vernetzten, teilweise neutralisierten, Hydrogel bildenden polymerischen Geliermittel-Materials umfaßt, bei welcher
(a) das genannte polymerische Geliermittel-Material umfaßt:
(i) von 50 Molprozent bis 99.999 Molprozent polymerisierte ungesättigte, polymerisierbare, Säuregruppeenthaltende Monomere; und
(ii) von 0.001 Molprozent bis 5 Molprozent einen vernetzenden Wirkstoff;
wobei das genannte polymerische Geliermittel-Material einen Neutralisationsgrad von mindestens 50 % und ein Gelvolumen von mindestens 20 Gramm synthetischen Urin pro Gramm Geliermittel-Materials aufweist; und
(b) die genannte Mehrzahl von Partikeln eine Massemittelwert-Partikelgröße im Bereich von 400 bis 700 Mikrometer mit nicht mehr als 16 Gew.-% der genannten Partikel mit einer Partikelgröße von weniger als 200 Mikrometer und nicht mehr als 16 Gew.-% der genannten Partikel mit einer Partikelgröße von mehr als 1200 Mikrometer aufweist.

12. Eine Zusammensetzung nach Anspruch 11, bei welcher das polymerische Geliermittel-Material aus Polyacrylaten und Acrylsäure-gepfropfter Stärke ausgewählt ist und in synthetischem Urin einen Gleichgewichtsgehalt an extrahierbarem Polymer von nicht mehr als 17 Gew.-% aufweist.

13. Eine Zusammensetzung nach Anspruch 12, bei welcher das genannte polymerische Geliermittel-Material ein Gelvolumen von 25 bis 60 Gramm synthetischen Urins pro Gramm Geliermittel aufweist.

14. Eine Zusammensetzung nach Anspruch 13, bei welcher die genannte Mehrzahl von Partikeln Geliermittels die folgende Partikelgrößenverteilung aufweist, wie sie durch die ASTM-Analyse auf U.S.Sieb-Serien-Gittern bestimmt worden ist:

| U.S. Sieb-Serien Alternative Bezeichnung | Verteilung (Gew-%) |
|---|---|
| Auf Nr. 14 | bis zu 0.1 |
| Auf Nr. 16 | bis zu 1.0 |
| Auf Nr. 20 | 10 - 35 |
| Auf Nr. 50 | weniger als 50 |
| Auf Nr.100 | bis zu 15 |
| Durch Nr.100 | weniger als oder gleich 5 |

## EP 0 304 319 B1

**Revendications**

1. Structure absorbant les liquides comprenant une bande ayant une densité allant de 0,06 à 0,3 g/cm$^3$, ladite bande comprenant de 40% à 95% en poids de libres hydrophiles et de 5% à 60% en poids de particules non fibreuses d'un agent gélifiant formant hydrogel dispersé parmi les libres hydrophiles de la bande, ledit agent gélifiant polymère ayant un volume de gel en équilibre d'au moins 20 grammes d'urine synthétique par gramme d'agent gélifiant,
caractérisée en ce que lesdites particules, lorsqu'elles sont dispersées parmi les libres hydrophiles de ladite bande, comme cela a été exposé ci-dessus, sont des particules discrètes, non agglomérées ou des particules agglomérées, non fragiles, lesdites particules ayant une dimension de particules globale, moyenne, de l'ordre de 400 à 700 micromètres, pas plus de 16% en poids de ces particules ayant une dimension de particule inférieure à 200 micromètres, et pas plus de 16% en poids de ces particules ayant une dimension de particule supérieure à 1200 micromètres, grâce à quoi la capacité absorbante effective en équilibre desdites particules, alors qu'elles sont dans ladite structure, est plus grande que la capacité absorbante effective en équilibre, correspondante des particules dans une structure comparative différant en composition et en structure de ladite première structure ci-dessus mentionnée seulement en ce que, les particules dans ladite structure comparative ont une dimension de particule globale, moyenne inférieure à 400 micromètres.

2. Structure absorbant les liquides selon la revendication 1, caractérisée en ce que lesdites particules non fibreuses de l'agent gélifiant, lorsqu'elles sont dispersées dans la bande parmi les fibres hydrophiles,
   (a) ont une dimension de particule globale, moyenne, de l'ordre de 410 à 650 micromètres, pas plus de 16% en poids de ces particules ayant une dimension de particule inférieure à 210 micromètres et pas plus de 16% en poids ayant une dimension de particule supérieure à 1000 micromètres; ou
   (b) ont une dimension de particule globale, moyenne, de l'ordre de 420 à 600 micromètres, pas plus de 16% en poids des particules ayant une dimension de particule inférieure à 220 micromètres et pas plus de 16% en poids ayant une dimension de particule supérieure à 900 micromètres.

3. Structure absorbant les liquides selon l'une quelconque des revendications 1 et 2, caractérisée en ce que ladite bande comprend de 70% à 90% en poids desdites fibres hydrophiles et de 10% à 30% en poids desdites particules non fibreuses d'agent gélifiant.

4. Structure absorbant les liquides selon l'une quelconque des revendications 1 à 3, caractérisée en ce que les fibres hydrophiles sont des fibres cellulosiques.

5. Structure absorbant les liquides selon l'une quelconque des revendications 1 à 4, caractérisée en ce que les fibres hydrophiles sont des fibres en pulpe de bois et l'agent gélifiant polymère formant hydrogel a un volume de gel de 25 à 60 grammes d'urine synthétique par gramme d'agent gélifiant.

6. Structure absorbant les liquides selon l'une quelconque des revendications 1 à 4, caractérisée en ce que les fibres hydrophiles sont des fibres de cellulose raidies, torsadées, enroulées, et en ce que l'agent gélifiant polymère formant hydrogel a un volume de gel allant de 20 à 35 grammes d'urine synthétique par gramme d'agent gélifiant.

7. Structure absorbant les liquides selon l'une quelconque des revendications 1 à 6, caractérisée en ce que l'agent gélifiant polymère formant hydrogel est un polymère légèrement réticulé, partiellement neutralisé choisi à partir d'amidon greffé de polyacrylates et d'acide acrylique, ledit agent gélifiant ayant une teneur en polymère en équilibre susceptible d'être extraite en urine synthétique qui n'est pas supérieure à 17% en poids.

8. Article absorbant les liquides comprenant:
   (a) une feuille de fond imperméable aux liquides;
   (b) une feuille de dessus perméable aux liquides; et
   (c) une âme absorbant les liquides disposée entre ladite feuille de fond et ladite feuille de dessus,
caractérisé en ce que ladite âme absorbante comprend une structure absorbant les liquides selon l'une quelconque des revendications 1 à 7.

18

EP 0 304 319 B1

9. Procédé pour fabriquer une structure absorbant les liquides comprenant une bande ayant une densité allant de 0,06 à 0,3 g/cm³, ladite bande comprenant de 40% à 95% en poids de fibres hydrophiles et de 5% à 60% en poids de particules non fibreuses d'un agent gélifiant polymère formant hydrogel ayant un volume de gel en équilibre d'au moins 20 grammes d'urine synthétique par gramme d'agent gélifiant, ledit procédé comprenant l'étape dans laquelle les particules dudit agent gélifiant sont fournies, à partir d'une source qui est périodiquement réalimentée, de manière à être étroitement dispersées à travers lesdites fibres hydrophiles, et ladite bande est étirée à partir du mélange étroit résultant de fibres et de particules; caractérisé en ce que le procédé comprend le contrôle de l'alimentation desdites particules à partir de ladite source, de telle sorte que les particules soient des particules discrètes, non agglomérées ou des particules agglomérées, non fragiles ayant une dimension de particule moyenne de l'ordre de 400 à 700 micromètres, pas plus de 16% en poids de ces particules ayant une dimension de particule inférieure à 200 micromètres et pas plus de 16% en poids de ces particules ayant une dimension de particule supérieure à 1200 micromètres, grâce à quoi la capacité absorbante effective, en équilibre desdites particules lorsqu'elles sont dans ladite structure est supérieure à la capacité absorbante en équilibre correspondante des particules dans une structure comparative différant en composition et en structure de ladite première structure ci-dessus mentionnée seulement en ce que, les particules dans ladite structure comparative ont une dimension de particule globale, moyenne inférieure à 400 micromètres.

10. Procédé selon la revendication 9, caractérisé en ce que l'alimentation desdites particules non fibreuses d'agent gélifiant à partir de ladite source est réglée de telle sorte que lesdites particules:
(a)ont une dimension de particule globale, moyenne, de l'ordre de 410 à 650 micromètres, pas plus de 16% en poids de ces particules ayant une dimension de particule inférieure à 210 micromètres et pas plus de 16% en poids ayant une dimension de particule supérieure à 1000 micromètres; ou
(b)ont une dimension de particule globale, moyenne, de l'ordre de 420 à 600 micromètres, pas plus de 16% en poids de particules ayant une dimension de particule inférieure à 220 micromètres et pas plus de 16% en poids ayant une dimension de particule supérieure à 900 micromètres.

11. Composition particulièrement appropriée pour être utilisée comme agent gélifiant absorbant dans des structures en bande, fibreuses, absorbantes, ladite composition comprenant une pluralité de particules non fibreuses, discrètes, non agglomérées ou de particules agglomérées, non fragiles d'un matériau d'agent gélifiant polymère formant hydrogel, partiellement neutralisé, légèrement réticulé, et pratiquement insoluble dans l'eau, dans laquelle:
(a)ledit matériau d'agent gélifiant polymère comporte:
(i) de 50 moles pour cent à 99,999 moles pour cent de monomères polymérisés non saturés, polymérisables contenant un groupe acide; et
(ii) de 0,001 mole pour cent à 5 moles pour cent d'un agent de réticulation;
ledit matériau d'agent gélifiant polymère ayant un degré de neutralisation d'au moins 50% et un volume de gel d'au moins 20 grammes d'urine synthétique par gramme de matériau d'agent gélifiant; et
(b)ladite pluralité de particules a une dimension de particule globale, moyenne de l'ordre de 400 à 700 micromètres, pas plus de 16% en poids desdites particules ayant une dimension de particule inférieure à 200 micromètres et pas plus de 16% en poids desdites particules ayant une dimension de particule supérieure à 1200 micromètres.

12. Composition selon la revendication 11, dans laquelle le matériau d'agent gélifiant polymère est choisi à partir d'amidon greffé de polyacrylates et d'acide acrylique et présente une teneur en polymère en équilibre susceptible d'être extraite dans l'urine synthétique qui n'est pas supérieure à 17% en poids.

13. Composition selon la revendication 12 dans laquelle ledit matériau d'agent gélifiant polymère a un volume de gel allant de 25 à 60 grammes d'urine synthétique par gramme d'agent gélifiant.

14. Composition selon la revendication 13 dans laquelle ladite pluralité de particules d'agent gélifiant a la distribution suivante de dimension de particule telle que déterminée par l'analyse ASTM sur les tamis à mailles de séries U.S.

19

| Séries de tamis U.S. Désignation des variantes | Distribution (% en poids) |
|---|---|
| Sur le n° 14 | Jusqu'à 0,1 |
| Sur le n° 16 | Jusqu'à 1,0 |
| Sur le n° 20 | 10-35 |
| Sur le n° 50 | Inférieure à 50 |
| Sur le n° 100 | Jusqu'à 15 |
| A travers le n° 100 | Inférieure ou égale à 5 |